(19) Europäisches Patentamt / European Patent Office / Office européen des brevets

(11) **EP 1 770 082 B1**

(12) **EUROPEAN PATENT SPECIFICATION**

(45) Date of publication and mention
of the grant of the patent:
**17.12.2008 Bulletin 2008/51**

(51) Int Cl.:
*C07C 67/58* *(2006.01)*

(21) Application number: **06020281.9**

(22) Date of filing: **27.09.2006**

(54) **Process for producing sorbitan fatty acid ester**

Verfahren zur Herstellung von Sorbitan-Fettsäureester

Procédé de préparation d'un ester d'acide gras de sorbitan

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **30.09.2005 JP 2005285971**

(43) Date of publication of application:
**04.04.2007 Bulletin 2007/14**

(73) Proprietor: **RIKEN VITAMIN CO., LTD.**
**Chiyoda-ku,**
**Tokyo 101-8370 (JP)**

(72) Inventors:
• **Nagahiro, Shinya**
**c/o Riken Vitamin Co.,Ltd**
**573-0065, Osaka (JP)**
• **Kamiryou, Eiji**
**c/o Riken Vitamin Co.,Ltd**
**573-0065, Osaka (JP)**

(74) Representative: **Albrecht, Thomas**
**Kraus & Weisert**
**Patent- und Rechtsanwälte**
**Thomas-Wimmer-Ring 15**
**80539 München (DE)**

(56) References cited:
**US-A- 5 306 831**

**Description**

TECHNICAL FIELD

The present invention relates to a process for producing a sorbitan fatty acid ester.

BACKGROUND ART

Sorbitan fatty acid esters are surfactants highly safe to use on human body and are widely used as a food additive and in the production of emulsions or other products in the field of cosmetics and pharmaceutical products. Several techniques for producing sorbitan fatty acid esters are known. One such technique involves direct esterification of sorbitol or an intramolecular condensate of sorbitol with fatty acids in the presence of acid or alkali catalysts (See, for example, Patent Documents 1, 2, 3, and 4). Another technique involves the use of lipase to esterify sorbitol intramolecular condensates with fatty acids (See, Patent Document 5). The direct esterification of sorbitol or an intramolecular condensate of sorbitol with fatty acids is currently employed in the industrial production of sorbitan fatty acid esters inside and outside Japan.

In carrying out the direct esterification of sorbitol or an intramolecular condensate of sorbitol with fatty acids, a monoester-rich reaction mixture can be obtained by reacting sorbitol or sorbitol intramolecular condensates with a fatty acid at a 1:1 molar ratio or higher ratio. However, considerable amounts of unreacted sorbitol or sorbitol intramolecular condensates inevitably remain in such a reaction mixture due to the low degree of esterification. Since sorbitol or sorbitol intramolecular condensates cannot be removed by vacuum distillation, commercially available sorbitan fatty acid ester products generally contain unreacted sorbitol or sorbitol intramolecular condensates.

Sorbitol or sorbitol intramolecular condensates are polyols and do not act as surfactants. Thus, sorbitan fatty acid ester products containing unreacted sorbitol or sorbitol intramolecular condensates have a decreased surfactant activity per unit weight. The products containing unreacted sorbitol or sorbitol intramolecular condensates are highly sticky and are difficult to handle.

Accordingly, techniques have been developed to separate unreacted sorbitol or sorbitol intramolecular condensates from the reaction mixture after completion of the reaction and thereby increase the sorbitan fatty acid ester content. In one such technique, small amounts of boric acid are added to a product obtained by esterification of a polyol with fatty acids and the mixture is stirred while being heated and is then filtered or left to stand to allow it to separate into two layers. In this manner, unreacted polyol and its condensate are removed (See, Patent Document 6). In another technique, a product obtained by esterification of sorbitol with fatty acids is dissolved in warm butanone to neutralize catalysts and the solution is extracted with water to remove unreacted polyol and neutralized salts (See, Patent Document 7).

Neither of these techniques is favorable, because in the former technique, boric acid remains in the product, whereas the organic solvents used in the latter technique add to the production cost and make the process unsafe. Thus, there is a need for a simple process for production of a sorbitan fatty acid ester that can effectively remove unreacted sorbitol or sorbitol intramolecular condensates.

Patent Document 1: Japanese Patent Publication No. Sho 44-2964
Patent Document 2: Japanese Patent Publication No. Sho 49-15246
Patent Document 3: Japanese Patent Laid-Open Publication No. Sho 57-45139
Patent Document 4: Japanese Patent Laid-Open Publication No. Sho 62-142141
Patent Document 5: Japanese Patent Laid-Open Publication No. Hei 8-173788
Patent Document 6: Japanese Patent Laid-Open Publication No. Sho 48-34113
Patent Document 7: Japanese Patent Laid-Open Publication No. Sho 50-46620 (Example 1)

DISCLOSURE OF THE INVENTION

PROBLEMS TO BE SOLVED BY THE INVENTION

Accordingly, it is an object of the present invention to provide an industrially practical process for producing a sorbitan fatty acid ester that can significantly reduce unreacted sorbitol or sorbitol intramolecular condensates present in the reaction mixture obtained by the direct esterification of sorbitol or sorbitol intramolecular condensates with fatty acids.

MEANS TO SOLVE THE INVENTION

In an effort to achieve the above-described object, the present inventors have conducted extensive studies and as a result have found that the objective can be achieved by adding glycerol to the reaction mixture obtained by the direct esterification of sorbitol or sorbitol intramolecular condensates with fatty acids, and separating/removing the glycerol phase containing the unreacted sorbitol or sorbitol intramolecular condensates to give the desired sorbitan fatty acid esters. It is this discovery that led to the present invention.
Specifically, the present invention comprises a process for producing a sorbitan fatty acid ester, comprising:

adding, at the temperature from 60°C to less than 180°C, glycerol to a reaction mixture obtained by direct esterification of sorbitol or sorbitol intramolecular condensates with a fatty acid; and,
subsequently separating/removing the glycerol phase containing unreacted sorbitol or sorbitol intramolecular condensates.

EFFECT OF THE INVENTION

According to the present invention, sorbitan fatty acid ester products containing a decreased amount of unreacted sorbitol or sorbitol intramolecular condensates can be produced in a simple and effective manner without using organic solvents.
Containing a decreased amount of unreacted sorbitol or sorbitol intramolecular condensates, the sorbitan fatty acid ester obtained by the process of the present invention has an improved surfactant activity per unit weight.
In addition, when the sorbitan fatty acid esters contain, for example, stearic acid as the major constituent fatty acid, they can be made into smooth powders that are easy to handle without non-sticky.

BEST MODE FOR CARRYING OUT THE INVENTION

Sorbitol for use in the present invention may be D-sorbitol in the form of white powder or granules or D-sorbitol solution containing approximately 50.0 to 70.0 mass% of D-sorbitol.
Sorbitol intramolecular condensates for use in the present invention are compounds obtained by intramolecular dehydration of sorbitol. Examples thereof include 1,5-sorbitan, 1,4-sorbitan, 2,5-sorbitan, 3.6-sorbitan, and 1,4,3,6-sorbide. These sorbitol intramolecular condensates may be used either individually or in combination of two or more. In addition to these compounds, the sorbitol intramolecular condensates for use in the present invention may contain unreacted sorbitol.
Intramolecular dehydration of sorbitol is preferably carried out by heating sorbitol in the presence of an acid catalyst (e.g., concentrated sulfuric acid, p-toluenesulfonic acid, and so on) at approximately 110 to 150°C, and preferably at approximately 120°C, and removing the generated water under a reduced pressure of, for example, approximately 1.3 kPa. When the reaction should be terminated will be determined by measuring the hydroxyl value of the dehydrated condensate. Once the reaction is terminated, an aqueous sodium hydroxide solution, for example, is added to the dehydrated condensate to neutralize the acid catalyst. The neutralized reaction mixture is, then preferably filtered by adding a diatomite or other filter aids.
The fatty acid for use in the present invention may be any fatty acid, such as straight-chained or branched, saturated or unsaturated fatty acids having 6 to 24 carbon atoms. Specific examples include caproic acid, caprylic acid, capric acid, lauric acid, myristic acid, palmitic acid, stearic acid, behenic acid, lignoceric acid, oleic acid, elaidic acid, linoleic acid, linolenic acid, erucic acid, isostearic acid, 2-ethylhexyl acid, and condensed ricinoleic acid. These fatty acids may be used either individually or in combination of two or more.
Although the amount of the fatty acid used relative to sorbitol or sorbitol intramolecular condensates may vary depending on the desired degree of esterification, a smaller degree of esterification indicates a larger amount of unreacted sorbitol or sorbitol intramolecular condensates present in the reaction product. It is thus particularly effective when approximately 0.1 to 1 mol of fatty acid is used for 1 mol of sorbitol or sorbitol intramolecular condensates in the process of the present invention.
According to the present invention, the esterification of sorbitol or sorbitol intramolecular condensates with fatty acids may be carried out with or without acid or alkali catalysts. The use of alkali catalysts is particularly preferred. Examples of acid catalysts include concentrated sulfuric acid and p-toluenesulfonic acid. Examples of alkali catalysts include potassium hydroxide, sodium hydroxide, potassium carbonate, and sodium carbonate. The alkali catalysts are typically used in an amount of approximately 0.01 to 1.0 mass%, and preferably in an amount of approximately 0.05 to 0.5 mass%, with respect to the total amount of reactants (Dry weight).
The esterification is generally carried out in a common reaction vessel that is equipped with a stirrer, a heating jacket, a baffle, an inert gas inlet, a thermometer and a condenser-equipped moisture separator. Sorbitol or sorbitol intramolecular condensates, fatty acids, and catalysts are fed to the reaction vessel and are mixed and stirred. The reaction is then carried out under an inert gas atmosphere such as nitrogen and carbon dioxide while the water produced by the esterification is removed from the system and the reaction mixture is heated to a particular temperature for a particular period of time. The reaction temperature is typically in the range of approximately 180 to 260°C, and preferably in the range of approximately 200 to 250°C. The reaction is carried out under reduced or atmospheric pressure for a time period of approximately 0.5 to 15 hours, and preferably approximately 1 to 3 hours. In general, the reaction is terminated when the measured acid value of the reaction mixture is decreased to below approximately 10.

Once the esterification has been completed, the catalyst remaining in the reaction mixture is neutralized if any catalyst is used. When the esterification is performed at 200°C or higher temperatures, the catalyst is preferably neutralized after the reaction mixture is cooled to approximately 180 to 200°C. When the esterification is performed at 200°C or lower temperatures, the catalyst may be neutralized without cooling the reaction mixture. For example, when sodium hydroxide is used as an alkali catalyst and is neutralized with phosphoric acid (85 mass%), the phosphoric acid is added to the catalyst and the mixture is well stirred to neutralize the catalyst. The amount of the phosphoric acid (85 mass%) to be added is typically equal to, or greater than, the amount of phosphoric acid (85 mass%) determined by the neutralization equation (1) below divided by 0.85*. Preferably, the amount of the phosphoric acid (85 mass%) is preferably about twice to three times the amount of phosphoric acid (85 mass%) determined by the neutralization equation (1) below that is divided by 0.85. Once neutralized, the reaction mixture is left at the same temperature preferably for approximately 0.5 hours or longer, and more preferably for approximately 1 to 10 hours. Any unreacted sorbitol or sorbitol intramolecular condensates separating as the bottom layer, is removed.

* When the amount of sodium hydroxide used is 1.0 g, the amount becomes to be approximately 0.96 g.

$$3NaOH + H_3PO_4 \rightarrow Na_3PO_4 + 3H_2O \qquad (1)$$

Subsequently, the reaction mixture is kept at the temperature from approximately 60°C to less than 180°C, preferably at the temperature from approximately 120°C to less than 180°C, and more preferably at approximately 130 to 150°C. In doing so, the mixture is cooled if necessary. Glycerol is then added in an amount of approximately 0.5 to 10 times, and preferably approximately 0.5 times to 5 times the total amount of the sorbitol or sorbitol intramolecular condensates and the fatty acid initially used in the reaction. The reaction mixture and glycerol were thoroughly mixed and the resulting mixture is left at the same temperature for approximately 0.5 hours or longer, and preferably for approximately 1 to 10 hours. When the mixture separates into two layers, the bottom layer (glycerol phase containing the unreacted sorbitol or sorbitol intramolecular condensates) was removed or centrifuged to remove the glycerol phase containing the unreacted sorbitol or sorbitol intramolecular condensates. If the amount of the glycerol added to the reaction mixture is too small, then the unreacted sorbitol or sorbitol intramolecular condensates cannot be removed to a sufficient degree, whereas if the amount of glycerol is too large, the separation and removal of glycerol phase takes too long, resulting in a decreased productivity.

The reaction mixture purified by the above-described process is preferably distilled under reduced pressure to remove the remaining glycerol and, if necessary, is subjected to such processes as desalting, decolorization, and filtration, thereby to obtain a sorbitan fatty acid ester product which as much of the unreacted sorbitol or sorbitol intramolecular condensates has been reduced as possible.

EXAMPLES

The present invention will now be described in further detail with reference to examples, which are not intended to limit the scope of the invention in any way.

[Example 1]

260 g (approximately 1.0 mol) of D-sorbitol solution (Trade Name: Sorbit D-70, TOWA CHEMICAL INDUSTRY) were fed to a 1L four-necked flask equipped with a stirrer, a thermometer, a gas inlet, and a water separator. The solution was dehydrated under reduced pressure of approximately 400 Pa at 75°C for approximately 10 min. 282 g of stearic acid (Trade Name: Stearic acid 90, MIYOSHI OIL AND FAT) was added thereto along with 5 mL of 10 w/v% aqueous sodium hydroxide solution to serve as a catalyst. Esterification was then carried out at 220°C for approximately 3 hours under normal pressure in a nitrogen gas stream until the acid value is decreased to below 10. Subsequently, the reaction mixture was cooled to approximately 180°C, at which point 1.15 g of phosphoric acid (85 mass%) were added to neutralize the catalyst. The mixture was further cooled to approximately 150°C and 400 g of glycerol were added. The mixture was mixed uniformly and was left for approximately 1 hour at the same temperature. The separated glycerol phase (approximately 320 g) was removed. The resultant sorbitan fatty acid ester was distilled at approximately 160°C under reduced pressure of approximately 250 Pa to remove the remaining glycerol. Thus approximately 400 g of a sorbitan stearate (Test Product 1) was obtained. This product has an acid value of 3.2 and a hydroxyl value of 250.

[Comparative Example 1]

260 g (approximately 1.0 mol) of D-sorbitol solution (Trade Name: Sorbit D-70, TOWA CHEMICAL INDUSTRY) were fed to a 1L four-necked flask equipped with a stirrer, a thermometer, a gas inlet, and a water separator. The solution was dehydrated under reduced pressure of approximately 400 Pa at 75°C for approximately 10 min. 282 g of stearic acid (Trade Name: Stearic acid 90, MIYOSHI OIL AND FAT) was added thereto along with 5 mL of a 10 w/v% aqueous sodium hydroxide solution to serve as a catalyst. Esterification was then carried out at 220°C for approximately 3 hours under normal pressure in a nitrogen gas stream until the acid value is decreased to below 10. Subsequently, the reaction mixture was cooled to approximately 180°C, at which point 1.15 g of phosphoric acid (85 mass%) were added to neutralize the catalyst. The mixture was then left for approximately 1 hour at the same temperature. The separated bottom layer (approximately 10 g) was removed, and then approximately 435 g of a sorbitan stearate (Test Product 2) was obtained. This product has an acid value of 3.8 and a hydroxyl value of 360.

[Example 2]

241 g (approximately 1.0 mol) of sorbitan (Trade Name: Sorbitan 70, SANKO CHEMICAL) were fed to a 1L four-necked flask equipped with a stirrer, a thermometer, a gas inlet, and a water separator. The solution was dehydrated under reduced pressure of approximately 400 Pa at 75°C for approximately 10 min. 282 g of stearic acid (Trade Name: Stearic acid 90, MIYOSHI OIL AND FAT) was added thereto along with 5 mL of a 10 w/v% aqueous sodium hydroxide solution to serve as a catalyst. Esterification was then carried out at 220°C for approximately 4 hours under normal pressure in a nitrogen gas stream until the acid value is decreased to below 10. Subsequently, the reaction mixture was cooled to approximately 180°C, at which point 1.15 g of phosphoric acid (85 mass%) were added to neutralize the catalyst. The mixture was further cooled to approximately 150°C and 500 g of glycerol were added. The mixture was mixed until uniformly and was left for approximately 1 hour at the same temperature. The separated glycerol phase (approximately 400 g) was removed. The resultant sorbitan fatty acid ester was distilled at approximately 160°C under reduced pressure of approximately 250 Pa to remove the remaining glycerol. Thus, approximately 380 g of a sorbitan stearate (Test Product 3) was obtained. This product has an acid value of 5.8 and a hydroxyl value of 205.

[Comparative Example 2]

241 g (approximately 1.0 mol) of sorbitan (Trade Name: Sorbitan 70, SANKO CHEMICAL) were fed to a 1L four-necked flask equipped with a stirrer, a thermometer, a gas inlet, and a water separator. The solution was dehydrated under reduced pressure of approximately 400 Pa at 75°C for approximately 10 min. 282 g of stearic acid (Trade Name: Stearic acid 90, MIYOSHI OIL AND FAT) was added thereto along with 5 mL of a 10 w/v% aqueous sodium hydroxide solution to serve as a catalyst. Esterification was then carried out at 220°C for approximately 4 hours under normal pressure in a nitrogen gas stream until the acid value is decreased to below 10. Subsequently, the reaction mixture was cooled to approximately 180°C, at which point 1.15 g of phosphoric acid (85 mass%) were added to neutralize the catalyst. The mixture was then left for approximately 1 hour at the same temperature. The separated bottom layer (approximately 10 g) was removed, and then approximately 415 g of a sorbitan stearate (Test Product 4) was obtained. This product has an acid value of 6.2 and a hydroxyl value of 270.

[Test Example 1]

Each of the sorbitan fatty acid esters (Test Products 1 to 4) obtained in Examples 1 and 2 and Comparative Examples 1 and 2 was analyzed for the amount of free polyols contained. The term "polyols" as used herein refers to alcohols that have two or more hydroxyl groups within their molecules. Examples are sorbitol, sorbitol intramolecular condensates (for example, 1,5-sorbitan, 1,4-sorbitan, 2,5-sorbitan, 3,6-sorbitan, and 1,4,3,6-sorbide) and glycerol. The results were shown in Table 1.

[Quantification]

A glass column (length = 21 cm, diameter = 2 cm) was packed with approximately 30 g of a reversed-phase silica gel (Trade Name: Inertsil ODS-3, GL SCIENCES) by a dry method. Approximately 10 g of each sample were accurately weighed and were dissolved in 50 mL of 25v/v% aqueous methanol. The solution was poured on the column, followed by 200 mL of 25v/v% aqueous methanol at a rate of 1 mL/1 min. The eluate was collected and was placed in a concentration flask of known weight. Using a rotary evaporator, the eluate was concentrated at approximately 90°C and approximately 4 kPa. The concentrate was allowed to cool in a desiccator and the total weight was accurately measured to determine the free polyol content (mass%) by the following equation.
$$\text{Free Polyol Content} = \frac{\text{Total weight(g)} - \text{Flask weight(g)}}{\text{Sample weight(g)}} \times 100(\text{mass\%})$$

(Table 1)

|  |  | Free Polyol Content (mass%) |
|---|---|---|
| Example | Test Product 1 | 2.2 |
|  | Test Product 3 | 1.3 |
| Comparative Example | Test Product 2 | 9.8 |
|  | Test Product 4 | 8.0 |

[Test Example 2]

Each of the sorbitan fatty acid esters (Test Products 1 to 4) obtained in Examples 1 and 2, and Comparative Examples 1 and 2 were subjected to a spray cooling and the resulting powder was observed. The results were shown in Table 2.

[Procedure]

A laboratory spray drier (Model L-8i, OHKAWARA KAKOHKI) was used. 200 g of each sample were melted at approximately 80°C and the molten product was sprayed through a pressure nozzle into the tower, which was conditioned to approximately 5 to 10°C by injecting liquid nitrogen. The cooled and solidified powder was recovered at the bottom of the tower.

(Table 2)

|  |  | State of Powder |
|---|---|---|
| Example | Test Product 1 | Smooth and in excellent condition |
|  | Test Product 3 | Smooth and in excellent condition |
| Comparative Example | Test Product 2 | Sticky and can be pressed into shape |
|  | Test Product 4 | Sticky and can be pressed into shape |

Claims

**1.** A process for producing a sorbitan fatty acid ester, **characterized in that** the process comprises:

adding, at the temperature from 60°C to less than 180°C, glycerol to a reaction mixture obtained by direct esterification of sorbitol or sorbitol intramolecular condensates with a fatty acid; and
subsequently separating/removing the glycerol phase containing unreacted sorbitol or sorbitol intramolecular condensates.

Patentansprüche

**1.** Verfahren zur Herstellung eines Sorbitanfettsäureesters, **dadurch gekennzeichnet, dass** das Verfahren umfasst:

Zugeben von Glycerin bei einer Temperatur von 60°C bis weniger als 180°C zu einem Reaktionsgemisch, das durch direkte Veresterung von Sorbit oder intramolekularen Sorbitkondensaten mit einer Fettsäure erhalten wurde, und
anschließendes Abtrennen/Entfernen der Glycerinphase, die nicht umgesetztes Sorbit oder nicht umgesetzte intramolekulare Sorbitkondensate enthält.

**Revendications**

1. Un procédé de préparation d'un ester d'acide gras de sorbitane **caractérisé en ce que** le procédé comprend les étapes consistant à

   - ajouter du glycérol, à une température d'au moins 60 °C et inférieure à 180 °C, à un mélange réactionnel obtenu par estérification directe de la sorbite ou des condensats intramoléculaires de la sorbite avec un acide gras ; et
   - puis séparer/éliminer la phase de glycérol qui contient de la sorbite non-réagie ou des condensats intramoléculaires non-réagis de la sorbite.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO442964 A
- JP SHO4915246 A
- JP SHO5745139 A
- JP SHO62142141 A
- JP HEI8173788 A
- JP SHO4834113 A
- JP SHO5046620 A